# EUROPÄISCHE PATENTSCHRIFT

(11) **EP 4 048 955 B1**
(45) Veröffentlichungstag und Bekanntmachung des Hinweises auf die Patenterteilung: **11.03.2026**
(21) Anmeldenummer: 20788731.6
(22) Anmeldetag: 01.10.2020
(51) Int. Cl.: F24C 15/20, B03C 3/09, A61L 9/22, B01D 46/00, B01D 46/12, B03C 3/08, B03C 3/12, B03C 3/41, B03C 3/47, B03C 3/60, F24F 8/192

(54) **DUNSTABZUGSHAUBE MIT ELEKTROSTATISCHER FILTEREINHEIT**
EXHAUST HOOD WITH ELECTROSTATIC FILTER UNIT
HOTTE ASPIRANTE AVEC UNITÉ DE FILTRATION ÉLECTROSTATIQUE

(30) Priorität: 23.10.2019 DE 102019216344
(43) Veröffentlichungstag der Anmeldung: 31.08.2022
(73) Patentinhaber: BSH Hausgeräte GmbH, 81739 München (DE)
(72) Erfinder: HEPPERLE, Georg, 74081 Heilbronn (DE); VOLLMAR, Daniel, 76327 Pfinztal (DE)
(86) Internationale Anmeldenummer: PCT/EP2020/077528
(87) Internationale Veröffentlichungsnummer: WO 2021/078489

(56) Entgegenhaltungen:
- CN-B- 106 733 194
- CN-U- 202 630 184
- CN-U- 208 066 543
- CN-U- 208 161 831
- DE-A1- 102017 214 495
- US-A1- 2005 109 204
- US-A1- 2008 170 971
- US-A1- 2009 010 801

## Beschreibung

Die Erfindung betrifft eine Dunstabzugshaube mit mindestens einer elektrostatische Filtereinheit.

In der US 2008/170971 A1 ist eine Luftreinigungsvorrichtung beschrieben, die einen lonisator, einen Photokatalysator, eine UV-Lichtquelle und mindestens einen porösen elektrostatischen Filter stromabwärts des lonisiators umfasst. Die Luftreinigungsvorrichtung kann einen Vorfilter umfassen, der einen elektrostatischen Filter oder einen mechanischen Filter darstellen kann.

In der US 2009/010801 A1 ist ein Luftreiniger beschrieben, der ein Gehäuse, einen katalytischen Reaktor aufweist. Vor dem katalytischen Reaktor kann ein elektrostatischer Abscheider angeordnet sein. Zudem kann ein Vorfilter vorgesehen sein, der große, sichtbare Partikel ausfiltert.

In der CN 106 733 194 B sind eine elektrostatische Vorrichtung und eine Dunstabzugshaube beschrieben. Die Vorrichtung umfasst ein elektrostatisches Sammelmodul, ein elektrostatisches Nadelmodul und ein elektrostatisches Ionisationsmodul, die in Reihe gestapelt sind.

In der CN 208 066 543 U wird eine elektrostatische Dampfreinigungsanlage für Küchenutensilien beschrieben. Die Vorrichtung umfasst eine Haube und einen in Strömungsrichtung des Dampfes angeordneten ersten Filter, einen zweiten Filter, eine UV-Lampe, eine elektrostatische Ionisationseinheit, eine elektrostatische Adsorptionseinheit und einen dritten Filter.

In der CN 202 630 184 U wird eine Art Abzugshaube mit statischem Rauchabscheider beschrieben. Die Vorrichtung umfasst eine Ölrauch-Abscheidevorrichtung, die eine Anordnung aus drei Elektrodenplatten, welche in Strömungsrichtung des Rauchs gestapelt angeordnet sind, enthält.

In der CN 208 161 831 U wird eine Plattenelektrodenvorrichtung für die Reinigung von Küchenabgasen beschrieben. Die Vorrichtung umfasst eine Ionisationszone und eine Adsorptionszone, wobei die negative Elektrodenplatte eine Blattstruktur und die positive Elektrodenplatte eine Netzstruktur aufweist. Die negative und die positive Platte befinden sich in der Adsorptionszone. Die Ionisationszone befindet sich am Anfang der Adsorptionszone.

Luftreinigungsvorrichtungen können beispielsweise Luftreiniger zum Filtern von Raumluft, Vorrichtungen zum Filtern von in einer Fahrgast-Kabine im Automobilbereich angesaugter Luft oder Dunstabzüge für Küchen, die beispielsweise Dunstabzugshauben darstellen, sein. Bei diesen Luftreinigungsvorrichtungen ist es bekannt, flüssige und feste Verunreinigungen sowie Gerüche aus der verunreinigten Luft oder den beim Kochen entstehenden Dünsten und Wrasen auszufiltern. Hierzu werden meist mechanische Filter eingesetzt. Als mechanische Filter werden beispielsweise Streckmetallfilter, Lochblechfilter, Baffle-Filter, die auch als Wirbelstromfilter bezeichnet werden können, Randabsaugungsfilter und poröse Schaumstoffmedien verwendet. Alle diese genannten Filtermedien filtern nach mechanischen Abscheidemechanismen wie dem Diffusionseffekt, Sperreffekt und maßgebend dem Trägheitseffekt.

Bei der Abscheidung nach dem Trägheitseffekt kann das Partikel aufgrund seiner Masseträgheit der Stromline des Gases (Luft) um die einzelnen Filterfasern, Streckmetalllagen, porösen Medien oder Ähnliches nicht folgen und kollidiert infolge dessen mit diesen. In Hinblick auf den dominierenden Trägheitseffekt steigt die Auftreffwahrscheinlichkeit eines Partikels auf die einzelne Faser des Filtermediums (entspricht der Abscheideeffizienz im Ganzen) mit steigender Partikelgeschwindigkeit, größer werdendem Partikeldurchmesser, steigender Filter-Packungsdichte und Filterdicken in Strömungsrichtung sowie kleiner werdendem Filterfaser-Durchmesser des Filtermediums.

Ein Nachteil dieser Filtereinheiten besteht darin, dass insbesondere eine hohe Anströmgeschwindigkeit angestrebt werden muss, um eine zufriedenstellende Filtereffizienz für Partikeldurchmesser größer 1 µm zu gewährleisten.

Zudem ist beispielsweise aus der DE 2146288 A eine Dunstabzugshaube bekannt, bei der eine elektrostatische Filtereinheit verwendet wird. Die elektrostatische Filtereinheit besteht bei dieser Dunstabzugshaube aus plattenförmigen Abscheide- und Gegenelektroden sowie drahtförmigen Ionisationselektroden. Die plattenförmigen Abscheide- und Gegenelektroden sind über elektrisch leitende Stege miteinander verbunden und sind so angeordnet, dass die in das Filterelement eintretende Luft zunächst die Abscheideelektroden mit dazwischen liegenden drahtförmigen Ionisationselementen anströmt und anschließend zu den nach oben versetzten Gegenelektroden gelangt. Die Abscheideelektroden sind über eine Trennwand an dem Gehäuse der Dunstabzugshaube befestigt. Zudem ist in dem Gehäuse der Dunstabzugshaube ein Hochspannungsgerät in der Nähe der Ansaugöffnung vorgesehen, das mit den Elektroden der Filtereinheit verbunden ist.

Ein Nachteil dieser Filtereinheit besteht darin, dass diese einen großen Bauraum in Anspruch nimmt und zugleich eine Gefahr für den Benutzer besteht.

Der Erfindung liegt daher die Aufgabe zugrunde eine Lösung zu schaffen, mittels derer bei einfachem Aufbau zuverlässig eine ausreichende Filtereffizienz gewährleistet werden kann und dennoch eine gefahrlose Benutzung gewährleistet ist.

Erfindungsgemäß wird die Aufgabe daher gelöst durch eine Dunstabzugshaube mit den Merkmalen des Anspruchs 1.

Die elektrostatische Filtereinheit wird im Folgenden auch als Filtereinheit oder als elektrostatischer Filter bezeichnet. Die Filtereinheit weist eine Ionisationseinheit und eine Abscheideeinheit auf. Die Ionisationseinheit kann auch als Ionisationsbereich bezeichnet werden und die Abscheideeinheit als Abscheidebereich. Um eine elektrostatische Abscheidung von Partikeln zu ermöglichen, welche sich in der Luft befinden, müssen diese zunächst elektrostatisch aufgeladen (ionisiert) werden. Hierzu dient die Ionisationseinheit. Die Abscheideeinheit ist der Ionisationseinheit in Strömungsrichtung nachgeschaltet. Die Ionisationseinheit weist vorzugsweise mindestens eine Ionisationselektrode und mindestens eine Gegenelektrode auf. Die Ionisationselektrode wird mit Spannung, vorzugsweise Hochspannung, beaufschlagt. Beim Durchströmen von verunreinigter Luft durch die Ionisationseinheit werden feste und flüssige Partikel elektrostatisch mittels der Ionisationselektrode, die auch als Sprühelektrode bezeichnet werden kann, mittels Koronaentladung elektrisch aufgeladen. Die Ionisationselektrode, die eine Draht-Ionisationselektrode darstellen kann, ist in der Ionisationseinheit vorzugsweise zwischen zwei plattenförmigen Gegenelektroden angeordnet. Dies ist notwendig, da die Partikel für gewöhnlich in ihrem ursprünglichen Zustand keine beziehungsweise für eine effiziente elektrostatische Abscheidung ungenügende elektrische Ladung aufweisen. Ziel der Ionisationseinheit ist die elektrische Partikelaufladung jedes einzelnen Partikels bis zu seiner maximalen elektrischen Sättigungsladung.

Die Abscheideeinheit umfasst zumindest zwei Niederschlagselektroden. Die mindestens zwei Niederschlagselektroden sind vorzugsweise parallel zueinander angeordnet. Die mindestens zwei Niederschlagselektroden stehen unter elektrischer Hochspannung und bauen dadurch ein elektrisches Feld zueinander auf. Hierbei ist mindestens eine Niederschlagselektrode eine negative Niederschlagselektrode und mindestens eine positive Niederschlagselektrode. Die Höhe beziehungsweise der Betrag der elektrischen Feldstärke ist dabei maßgebend abhängig vom elektrischen Potential, das heißt von dem Betrag der Spannung in kV, dem Abstand der positiven und negativen Niederschlagselektroden zueinander sowie der geometrischen Form der Niederschlagselektroden.

Die aus der Ionisationseinheit austretende Luft mit den elektrisch geladenen Partikeln strömt in die Abscheideeinheit. Aufgrund des dort zwischen den Niederschlagselektroden aufgebauten elektrischen Feldes werden die Partikel an den Niederschlagselektroden abgeschieden und damit aus der Luft ausgefiltert.

Für die Ionisation der Partikel aus der Luft, als auch deren Abscheidung, ist eine elektrische Hochspannung von mehreren tausend Volt notwendig, dabei kann sowohl positive als auch negative Hochspannung Anwendung finden. Für die Generierung/Erzeugung dieser notwendigen elektrischen Hochspannung wird ein Hochspannungsübertrager, der auch als Hochspannungserzeuger, Hochspannungsnetzteil bezeichnet werden kann, eingesetzt. Dieser Hochspannungsübertrager versorgt den Ionisationsbereich und den Abscheidebereich der elektrostatischen Filterkassette mit elektrischer Hochspannung beziehungsweise elektrischer Energie. Der Hochspannungsübertrager ist dabei vorzugsweise in das die elektrostatische Filtereinheit, die auch als Filtermodul oder Filterkassette bezeichnet werden kann, implementiert. Das elektrostatische Filtermodul/Filterkassette ist vorzugsweise im Lufteinsaugbereich des Dunstabzugs angeordnet, um die dahinterliegenden Komponenten desgleichen vor Kochwrasen/Aerosolen/Schmutz zu schützen. Jedoch kann eine solche Filtervorrichtung optional auch im Luftausblasbereich im Dunstanzug angeordnet werden oder entlang der Luftströmungsführung zwischen dem Einlass- und Auslassbereich des Dunstabzugs.

Erfindungsgemäß sind die Niederschlagselektroden luftdurchlässig. Erfindungsgemäß bestehen die Niederschlagselektroden aus einem luftdurchlässigen Material. In diesem Fall werden die Niederschlagselektroden auch als poröse Niederschlagselektroden bezeichnet. Die Niederschlagselektroden können alle aus dem gleichen luftdurchlässigen Material bestehen. Es liegt aber auch im Rahmen der Erfindung, dass verschiedene Niederschlagselektroden aus unterschiedlichen Materialien bestehen. Der Vorteil der Verwendung von luftdurchlässigem Material für die Niederschlagselektroden besteht darin, dass zum einen die Herstellung des elektrostatischen Filters erleichtert ist, da die geforderte Luftdurchlässigkeit durch das Material selber gegeben ist. Zum anderen weisen bei einem luftdurchlässigen Material die Öffnungen in dem Material eine geringe Größe auf, wodurch ein effizientes Abscheiden von Partikeln aufgrund des mechanischen Abscheideeffekts gewährleistet werden kann. Gemäß einer nicht-erfindungsgemäßen Ausführungsform, bei der die Niederschlagselektrode aus einem luftundurchlässigen Material mit mindestens einer Luftdurchlassöffnung bestehen, ist es auch möglich, dass nur einige der Niederschlagselektroden, beispielsweise nur die positiven oder nur die negativen Niederschlagselektroden aus einem solchen Material bestehen und die jeweils anderen Niederschlagselektroden aus luftdurchlässigem Material bestehen. Weiterhin ist es auch möglich, dass beispielsweise nur die erste, das heißt der Ionisierungseinheit zugewandte, Niederschlagselektrode aus einem luftundurchlässigen Material mit Luftdurchlassöffnungen besteht. Das luftundurchlässige Material kann beispielsweise ein Blech sein. Die Luftdurchlassöffnungen können beispielsweise Löcher sein, die in das Blech gestanzt oder auf andere Art eingebracht werden. Insbesondere kann das luftundurchlässige Material mit Luftdurchlassöffnungen Streckmetall sein.

Das Material der mindestens einen Niederschlagselektrode kann daher beispielsweise Streckmetall, Drahtgewebe, Faserwerkstoff, Vlies, Sinterkunststoff oder Schaumstoff sein. Hierdurch wird eine Reihe von Vorteilen erzielt. Zum einen kann der Luftstrom nicht nur, wie im Stand der Technik an den Niederschlagselektroden entlang strömen, sondern kann durch diese hindurchströmen. Aufgrund der Luftdurchlässigkeit der Niederschlagselektroden können diese somit als mechanischer Filter dienen. Da die Abscheideeinheit der Ionisationseinheit in Strömungsrichtung nachgelagert ist, treten die in der Luft enthaltenen Partikel in einem elektrisch geladenen Zustand in die Abscheideeinheit ein. Somit wird die Abscheidung der Partikel an den Niederschlagselektroden sowohl durch die mechanischen Filtereffekte als auch durch die elektrische Ladung bewirkt, das heißt durch den elektrostatischen Filtereffekt erzielt.

Klassische, mechanische Filter haben die Eigenschaft, dass aufgrund des dominierenden Trägheitseffekts für Partikeldurchmesser >1 µm die Filtereffizienz mit steigender Strömungsgeschwindigkeit steigt. Bei reinem elektrostatischem Filter hingegen steigt die Filtereffizienz mit sinkender Strömungsgeschwindigkeit, weil die Verweilzeit des Partikels im lonisations- und Abscheidebereich damit zunimmt. Durch die Kombination der vorliegenden Erfindung werden die Vorteile sowohl der mechanischen als auch der elektrostatischen Filtermechanismen effizient ausgenutzt.

Zudem ist bei klassischen, elektrostatischen Filtern die Filtereffizienz signifikant vom Betrag der elektrischen Ionisations- und Abscheidespannung abhängig. Falls es zu einem Versagen der elektronischen Hochspannungskomponente (Spannungsausfall) kommt oder diese aufgrund von Kurzschlüssen ausfällt, ist gar keine Filterleistung mehr gegeben. Bei der vorliegenden Erfindung hingegen bleiben die mechanischen Filtermechanismen beziehungsweise Filtereffekte weiterhin erhalten. Ein Totalausfall der Gesamtfilterleistung tritt somit nicht ein.

Schließlich können aufgrund der Luftdurchlässigkeit der Niederschlagselektroden Partikel zumindest teilweise in den Poren oder anderen Luftdurchlassöffnungen der Niederschlagselektroden gehalten werden.

Die elektrostatische Filtereinheit weist erfindungsgemäß mindestens ein Schutzelement für zumindest eines der Elemente der Ionisationseinheit auf. Als Schutzelement wird im Sinne der Erfindung ein Element oder eine Vorrichtung bezeichnet, die den Benutzer vor einem direkten oder indirekten Kontakt mit einem der Elemente der Ionisationseinheit schützt und/oder die Elemente der Ionisationseinheit vor mechanischer Beschädigung schützt.

Indem mindestens ein solches Schutzelement vorgesehen ist, kann die elektrostatische Filtereinheit mit Hochspannung betrieben werden und dadurch die Steigerung der Filtereffizienz erzielt werden und der Benutzer sowie die Filtereinheit geschützt werden.

Gemäß einer Ausführungsform stellt das Schutzelement ein Schutzelement gegen Krafteinwirkung dar. Bei dieser Ausführungsform kann das Schutzelement vorzugsweise ein flächiges Element darstellen, das in Strömungsrichtung vor der Ionisationseinheit angeordnet ist. Das Schutzelement ist dabei luftdurchlässig.

Zusätzlich oder alternativ kann das Schutzelement ein Abschirmelement zur elektrischen Abschirmung sein. Insbesondere können die Ionisationseinheit und Abscheideeinheit, abgeschirmt werden.

Erfindungsgemäß stellt das Schutzelement eine Eingreifschutzvorrichtung darstellt, die mindestens eine Lage aufweist und der Ionisationseinheit in Strömungsrichtung vorgeschaltet ist. Indem das Schutzelement der Ionisationseinheit in Strömungsrichtung vorgeschaltet ist, kann sowohl der physische Kontakt zu der Ionisationseinheit durch den Benutzer als auch eine elektrische Abschirmung der Ionisationseinheit gegenüber dem Benutzer gewährleistet werden. Die Größe der Eingreifschutzvorrichtung ist vorzugsweise so bemessen, dass die Seite der Ionisationseinheit, die der Strömungsrichtung zugewandt ist, vollständig durch die Lage der Eingreifschutzvorrichtung abgedeckt ist.

Erfindungsgemäß stellt mindestens eine Lage der Eingreifschutzvorrichtung einen Vorfilter dar. Als Vorfilter wird eine Lage bezeichnet, die zum Filtern von relativ großen Schmutz- und Staubpartikel, insbesondere von Partikeln mit einem Durchmesser von d_{hyd} >= 1 µm dient. Indem das Schutzelement als Vorfilter ausgestaltet ist, kann neben der Verhinderung des Eingriffs des Benutzers in die Ionisationseinheit und vorzugsweise die elektrische Abschirmung der Ionisationseinheit sowie der Abscheideeinheit zusätzlich die Filtereffizienz der elektrostatischen Filtereinheit steigern. Der Vorfilter aus Streckmetall, perforiertem Blech, Drahtgewebe, Schweißgitter oder gewebtem Drahtgitter bestehen.

Gemäß einer Ausführungsform weist die Eingreifschutzvorrichtung zusätzlich zu dem Vorfilter mindestens eine Schutzlage und ein Distanzelement auf. Das Distanzelement ist dabei zwischen dem Vorfilter und der Schutzlage angeordnet. Die Schutzlage ist vorzugsweise die Lage, die in Strömungsrichtung die erste Lage der Eingreifschutzvorrichtung ist. Die Schutzlage kann aus einem elektrisch leitfähigen Material, insbesondere Metall, oder aus einem elektrisch isolierenden Material, insbesondere Kunststoff hergestellt sein.

Gemäß einer Ausführungsform besteht das Schutzelement zumindest teilweise aus einem elektrisch leitenden oder antistatisches Element mit einen Oberflächenwiderstand R <= 10¹¹ Ohm. Besonders bevorzugt besteht zumindest der Vorfilter aus einem solchen Material. In dem solches Material verwendet wird, kann Spannung, die in dem Vorfilter vorliegt zumindest abgeleitet werden.

Gemäß einer weiteren Ausführungsform stellt mindestens ein Schutzelement zumindest einen Teil eines Gehäuses für die Ionisationseinheit dar. Insbesondere stellt das Schutzelement in dieser Ausführungsform vorzugsweise zumindest einen Teil eines Isolierungsgehäuses der Ionisationseinheit dar. In dieser Ausführungsform ist das Schutzelement vorzugsweise aus einem isolierenden Material. Indem ein isolierendes Schutzelement vorgesehen ist, können insbesondere spannungsbeladene Elemente der Ionisationseinheit so aufgenommen werden, dass diese von dem Benutzer nicht erreicht werden können.

Gemäß einer Ausführungsform weist die Filtereinheit einen Hochspannungsübertrager auf und der Hochspannungsübertrager ist in einem der Gehäuseteile integriert. Hierdurch kann der Zugriff des Benutzers auf den Hochspannungsübertrager verhindert werden. Zudem kann durch die Integration des Hochspannungsübertragers in die Filtereinheit der Aufbau der Luftreinigungsvorrichtung vereinfacht werden, da diese lediglich Leitungen und Kontaktstellen für niedrige Spannung aufweisen müssen.

Gemäß einer bevorzugten Ausführungsform weist die Filtereinheit ein Schutzelement auf, das einen Vorfilter darstellt und der Vorfilter ist mit der Rückführung der Sekundärseite des Hochspannungsübertragers verbunden.

Gemäß einer Ausführungsform weist die elektrostatische Filtereinheit einen Filterrahmen auf und der Filterrahmen ist mit der Rückführung der Sekundärseite des Hochspannungsübertragers verbunden.

Erfindungsgemäß liegen die Niederschlagselektroden senkrecht zu der Strömungsrichtung der Luft durch die Ionisationseinheit. Die Strömungsrichtung der Luft durch die Ionisationseinheit ist vorzugsweise parallel zu der oder den Gegenelektroden der Ionisationseinheit. Somit liegen die Niederschlagselektroden vorzugsweise quer und insbesondere senkrecht zu der oder den Gegenelektroden der Ionisationseinheit.

Durch diese Ausrichtung der luftdurchlässigen Niederschlagselektroden kann der Luftstrom, der in die Abscheideeinheit eintritt, vollständig durch die Niederschlagselektroden geführt werden. Somit wird die Filtereffizienz weiter gesteigert. Zudem kann durch diese Ausrichtung der Niederschlagselektroden der Bauraum, der für die Filtereinheit erforderlich ist, minimiert werden. Im Gegensatz zu Filtereinheiten, bei denen die Niederschlagselektroden parallel zu dem Luftstrom und vorzugsweise parallel zu der oder den Gegenelektroden der Ionisationseinheit liegt, ist die Höhe oder Länge dieser Ausführungsform der erfindungsgemäßen Filtereinheit geringer, da in dieser Richtung die Niederschlagselektroden quer liegen.

Gemäß einer Ausführungsform liegen die Niederschlagselektroden aneinander an. Bei klassischen elektrostatischen Filtern mit Platten- oder Rohrabscheidern in der Abscheideeinheit, ist relativ betrachtet deutlich mehr Platzbedarf für den elektrostatischen Filter im Ganzen und speziell für den Abscheidebereich notwendig. Bei der erfindungsgemäßen Filtereinheit sind die Niederschlagselektroden luftdurchlässig. Vorzugsweise stellen die Niederschlagselektroden luftdurchlässige Platten oder Lagen dar. Somit ist auch beim Vorsehen mehrerer Niederschlagselektroden, die aufeinander aufliegen, die Gesamthöhe des Stapels an Niederschlagselektroden gering. Zudem kann durch den geringen Abstand der Niederschlagselektroden zueinander das zwischen den einzelnen luftdurchlässigen Niederschlagselektroden abgeschiedene / gefilterte Partikelgut aufgrund von Kapillarität zwischen den Niederschlagselektroden gehalten werden. Somit kann der erfindungsgemäße Abscheidebereich diese Partikel zusätzlich zu einer Speicherung in der Niederschlagselektrode selber speichern.

Die Reihenfolge der Niederschlagselektroden in der Abscheideeinheit kann erfindungsgemäß frei gewählt werden. So ist es beispielsweise möglich an der Seite der Abscheideeinheit, die der Ionisationseinheit zugewandt ist und über die Luft in die Abscheideeinheit eintritt, eine positive Niederschlagselektrode anzuordnen und anschließend alternierend jeweils negative und positive Niederschlagselektroden anzuordnen. Alternativ kann aber auch eine negative Niederschlagselektrode an der der Ionisationseinheit zugewandten Seite als erste Niederschlagselektrode angeordnet sein und anschließend alternierend positive und negative Niederschlagselektroden angeordnet sein.

Gemäß einer weiteren Ausführungsform ist es aber auch möglich, dass mindestens zwei zueinander benachbarte Niederschlagselektroden gleichgepolt sind. Somit können beispielsweise zwischen zwei positiven Niederschlagselektroden zwei oder mehr negative Niederschlagselektroden angeordnet sein.

Gemäß einer bevorzugten Ausführungsform weist mindestens eine der Niederschlagselektroden eine Isolierungsbeschichtung auf. Die Isolierungsbeschichtung kann mittels Pulverbeschichtung, Tauchlackierung oder einem anderen Beschichtungsverfahrens auf die Niederschlagselektroden aufgebracht sei / werden. Hierbei wird vorzugsweise die jeweilige Niederschlagselektrode vollständig elektrisch isoliert, wobei an der jeweils erforderlichen elektrischen Kontaktstelle zur Beaufschlagung der Niederschlagselektrode mit Spannung die Isolierungsbeschichtung ausgespart wird. Hierdurch können elektrische Kurzschlüsse und damit verbundene Spannungseinbrüche zwischen den einzelnen alternierenden unter Spannung stehenden Niederschlagselektroden vermieden werden.

Es können erfindungsgemäß alle Niederschlagselektroden der Abscheideeinheit mit einer Isolierungsbeschichtung versehen werden. Vorzugsweise werden jedoch nur die positiven Niederschlagselektroden elektrisch isoliert. Die Partikel werden in der Ionisationseinheit geladen. Trifft ein positiv geladenes Partikel auf eine negative Niederschlagselektrode auf, so soll dieses seine Ladung auch wieder abgeben kann, da sonst das elektrische Feld zwischen den Lagen dadurch mit der Zeit geschwächt wird. Indem aber bei der genannten Ausführungsform die positiven Niederschlagselektroden eine Isolierungsbeschichtung aufweisen, kann ein elektrischer Kurzschluss zwischen den positiven und negativen Niederschlagselektroden auch bei einem geringen Abstand oder einem Anliegen der Niederschlagselektroden aneinander verhindert werden.

Vorteile und Merkmale, die bezüglich der elektrostatischen Filtereinheit beschrieben werden, gelten - soweit anwendbar - entsprechend für die Dunstabzugshaube und umgekehrt.

Die Luftreinigungsvorrichtung kann beispielsweise ein Luftreiniger zum Filtern von Raumluft, eine Vorrichtung zum Filtern von in einer Fahrgast-Kabine im Automobilbereich angesaugter Luft oder einen Dunstabzug für Küchen sein. Die Luftreinigungsvorrichtung kann mehrere elektrostatische Filtereinheiten aufweisen. Die mindestens eine elektrostatische Filtereinheit ist vorzugsweise an der Ansaugseite der Luftreinigungsvorrichtung angeordnet. Es liegt aber auch im Rahmen der Erfindung zusätzlich oder alternativ mindestens eine elektrostatische Filtereinheit an der Luftauslassseite der Luftreinigungsvorrichtung vorzusehen.

Erfindungsgemäß stellt die Luftreinigungsvorrichtung eine Dunstabzugshaube dar.

Gemäß einer Ausführungsform weist die Dunstabzugshaube mindestens eine Kontaktstelle für den Kontakt mit einem Hochspannungsübertrager der Filtereinheit auf.

Die Erfindung wird im Folgenden erneut unter Bezugnahme auf die beiliegenden Figuren genauer beschrieben. Es zeigen:
- Figur 1:: eine schematische Perspektivansicht einer Ausführungsform einer erfindungsgemäßen Luftreinigungsvorrichtung;
- Figur 2:: eine schematische Explosionsdarstellung einer Ausführungsform einer erfindungsgemäßen elektrostatischen Filtereinheit;
- Figur 3:: eine schematische Explosionsdarstellung der Eingreifvorrichtung der elektrostatischen Filtereinheit nach Figur 2;
- Figur 4:: eine schematische Explosionsdarstellung der Ionisationseinheit der elektrostatischen Filtereinheit nach Figur 2;
- Figur 5:: eine schematische Explosionsdarstellung der Abscheideeinheit der elektrostatischen Filtereinheit nach Figur 2;
- Figur 6a und 6b:: schematische Perspektivansichten von Ausführungsformen der Niederschlagselektroden der elektrostatischen Filtereinheit nach Figur 2;
- Figur 7:: eine schematische Schnittansicht einer Ausführungsform der Niederschlagelektroden der erfindungsgemäßen elektrostatischen Filtereinheit;
- Figuren 8a und 8b:: schematische Schnittansichten weiterer Ausführungsformen der Niederschlagelektroden der erfindungsgemäßen elektrostatischen Filtereinheit;
- Figur 9:: eine schematische Darstellung der Verkabelung einer Ausführungsform der erfindungsgemäßen elektrostatischen Filtereinheit; und
- Figur 10:: eine schematische Diagrammdarstellung der Filtereffizienz der erfindungsgemäßen elektrostatischen Filtereinheit im Vergleich zu Streckmetallfiltern.

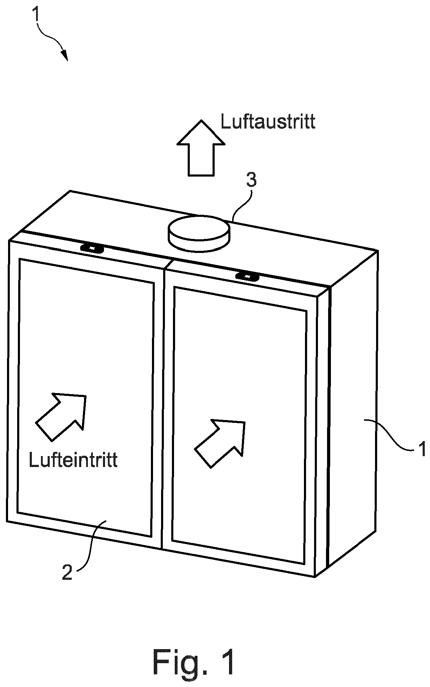

In Figur 1 ist eine Ausführungsform einer erfindungsgemäßen Luftreinigungsvorrichtung 1 gezeigt. In der gezeigten Ausführungsform stellt die Luftreinigungsvorrichtung 1 eine Dunstabzugshaube dar. In der Vorderseite der Luftreinigungsvorrichtung 1 ist eine Ansaugöffnung gebildet, in der in der gezeigten Ausführungsform zwei elektrostatische Filtereinheiten 2 angeordnet sind. Es liegt aber auch im Rahmen der Erfindung, dass nur eine oder mehr als zwei elektrostatischen Filtereinheiten 2 in der Ansaugöffnung angeordnet sind. In der Oberseite der Luftreinigungsvorrichtung ist ein Luftauslassstutzen 3 für den Luftaustritt aus der Luftreinigungsvorrichtung 1 vorgesehen. In der gezeigten Ausführungsform liegen die Ansaugöffnung und damit die elektrostatischen Filtereinheiten 2 in der Vertikalen. Es liegt aber auch im Rahmen der Erfindung, dass die Ansaugöffnung und damit die elektrostatischen Filtereinheiten 2 in einem beliebigen Anstellwinkel zwischen der Vertikalen und der Horizontalen liegen. Die elektrostatischen Filtereinheiten 2 weisen in der gezeigten Ausführungsform eine Kassettenform auf. Die elektrostatischen Filtereinheiten 2 werden daher auch als Filterkassetten bezeichnet. Insbesondere weisen die elektrostatischen Filtereinheiten 2 jeweils eine Breite und Länge auf, die größer ist als die Tiefe. Als Tiefe wird hierbei die Abmessung in der Hauptströmungsrichtung, die in der gezeigten Ausführungsform in der Horizontalen liegt, bezeichnet. Die Hauptströmungsrichtung ist in den Figuren durch einen Blockpfeil angedeutet. Die elektrostatischen Filtereinheiten 2 decken die Ansaugöffnung vollständig ab. Somit wird die gesamte, die Luftreinigungsvorrichtung 1 anströmende Luft durch die elektrostatischen Filtereinheiten 2 geleitet. Im Betrieb der Luftreinigungsvorrichtung strömt die verunreinigte Luft durch die großflächigen Filterflächen der elektrostatischen Filtereinheiten 2 und wird mittels dem elektrostatischen als auch den mechanischen Filtermechanismen von flüssigen und festen Verunreinigungen/Partikel, insbesondere mit einem hydraulischen Durchmesser D_{hyd} >= 0,001 µm durch Ausfiltern befreit. Wenn die elektrostatischen Filtereinheiten 2 ihren Sättigungsgrad an Verunreinigungen erreicht haben, können diese von der Luftreinigungsvorrichtung abgenommen und im Geschirrspüler oder manuell von Hand unter Wasser- und Reinigungsmitteleinwirkung gereinigt werden.

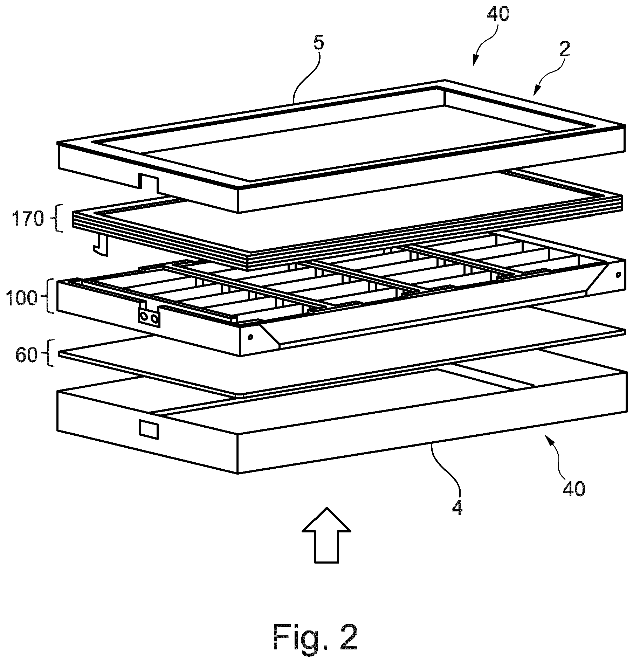

In Figur 2 ist eine schematische Explosionsdarstellung einer Ausführungsform einer erfindungsgemäßen elektrostatischen Filtereinheit 2 gezeigt. Die elektrostatische Filtereinheit 2 besteht in der gezeigten Ausführungsform aus einem Filterrahmen 40, einer Eingreifschutzvorrichtung 60, einer Ionisationseinheit 100 und einer Abscheideeinheit (170). Diese einzelnen Funktionsbaugruppen der elektrostatische Filtereinheit 2 weisen jeweils eine flächige Form auf beziehungsweise definieren eine Fläche. Der Filterrahmen 40 besteht in der gezeigten Ausführungsform aus zwei Teilen. Das in Figur 2 als unteres Teil gezeigte Teil wird im Folgenden als Filterrahmen Vorderseite 4 und das als oberes Teil gezeigte Teil des Filterrahmens 40 wird als Filterrahmen Rückseite 5 bezeichnet. Zur Herstellung beziehungsweise zur Montage der elektrostatischen Filtereinheit 2 können die Eingreifschutzvorrichtung 60, die Ionisationseinheit 100 und die Abscheideeinheit 170 in den Filterrahmen Vorderseite 4 gelegt und anschließend mit dem Filterrahmen Rückseite 5 verschlossen werden.

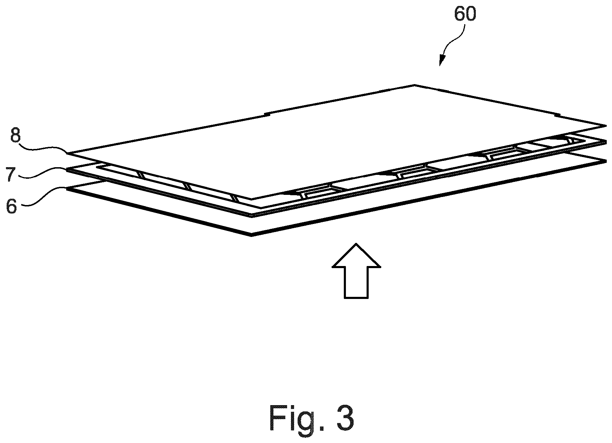

In Figur 3 ist die Ausführungsform der Eingreifschutzvorrichtung 60 in Explosionsdarstellung gezeigt. Die Eingreifschutzvorrichtung 60 bildet ein Schutzelement entsprechend der vorliegenden Erfindung. In der gezeigten Ausführungsform besteht die Eingreifschutzvorrichtung 60 aus drei Einzelteilen. Insbesondere besteht die Eingreifschutzvorrichtung 60 aus einer Schutzlage 6, einem Distanzelement Eingreifschutz 7 und einer Filterlage der Vorfilterung 8, die im Folgenden auch als Vorfilter bezeichnet wird. Die Einzelteile der Eingreifschutzvorrichtung 60 weisen jeweils eine flächige Form beziehungsweise Lagenform auf. Die erste Lage, die die eigentlichen Schutzlage 6 bildet, schützt die dahinterliegenden Einzelteile der Eingreifschutzvorrichtung 60 und Baugruppen der elektrostatischen Filtereinheit 2 vor unsachgemäßer Krafteinwirkung von außen. Zudem kann die Schutzlage 6 als Designelement zur Optik der gesamten Filterkassette beitragen. Die Schutzlage 6 kann aus einem elektrisch leitfähigen Material, insbesondere Metall, oder aus einem elektrisch isolierenden Material, insbesondere Kunststoff hergestellt sein.

Neben dem mechanischen Schutz gegen Krafteinwirkung kann die Eingreifschutzvorrichtung 60 auch als elektrisches Schutzelement dienen. Insbesondere die Filterlage zur Vorfilterung 8 dient als elektrisches Schutzelement. Die Filterlage zur Vorfilterung 8 dient zum einen als Vorfilter für relativ große Schmutz- und Staubpartikel, insbesondere für Partikel mit d_{hyd} >= 1 µm. Zum anderen dient die Filterlage der Vorfilterung 8 auch als elektrische Abschirmung. Hierbei erfolgt die Abschirmung durch die Filterlage der Vorfilterung 8 nach dem Prinzip eines Faraday'schen Käfigs. Insbesondere werden die Ionisationseinheit 100 und Abscheideeinheit 170, welche sich in Strömungsrichtung hinter der Filterlage der Vorfilterung 8 befinden durch diese abgeschirmt. Insbesondere bewirkt die Filterlage der Vorfilterung 8, dass der äußere Einsaugbereich, das heißt der Bereich, der in Strömungsrichtung vor der elektrostatischen Filtereinheit 2 liegt, infolge der statischen und quasistatischen elektrischen Felder der Ionisationseinheit feldfrei bleibt und die daraus resultierende elektrische Influenz nach außen, insbesondere außerhalb der Schutzlage 6 unterbunden wird. Ungewollte statische Aufladungen der Schutzlage 6 werden dadurch unterbunden. Die Filterlage der Vorfilterung 8 besteht vorzugsweise aus einem luftdurchlässigen elektrisch leitenden oder antistatisches Material. Beispielsweise kann die Filterlage der Vorfilterung aus elektrisch leitendem Kunststoff hergestellt sein. Die Filterlage der Vorfilterung 8 kann aber beispielsweise auch aus Streckmetall, perforiertem Blech, Drahtgewebe, Schweißgitter oder gewebtem Drahtgitter bestehen. Die Filterlage der Vorfilterung 8 weist vorzugsweise einen Oberflächenwiderstand R <=10¹¹ Ohm auf. In der gezeigten Ausführungsform besteht die Filterlage der Vorfilterung 8 aus nur einer Lage. Es liegt aber auch im Rahmen der Erfindung, dass die Filterlage der Vorfilterung 8 aus mehr als einer Lage besteht.

Zwischen der Filterlage der Vorfilterung 8 und der Schutzlage 6 ist eine weitere Lage der Eingreifschutzvorrichtung 60 vorgesehen. Diese wird als Distanzelement Eingreifschutz 7 bezeichnet und dient dazu die Filterlage der Vorfilterung 8 und der Schutzlage 6 auf einem definierten Abstand zueinander zu halten.

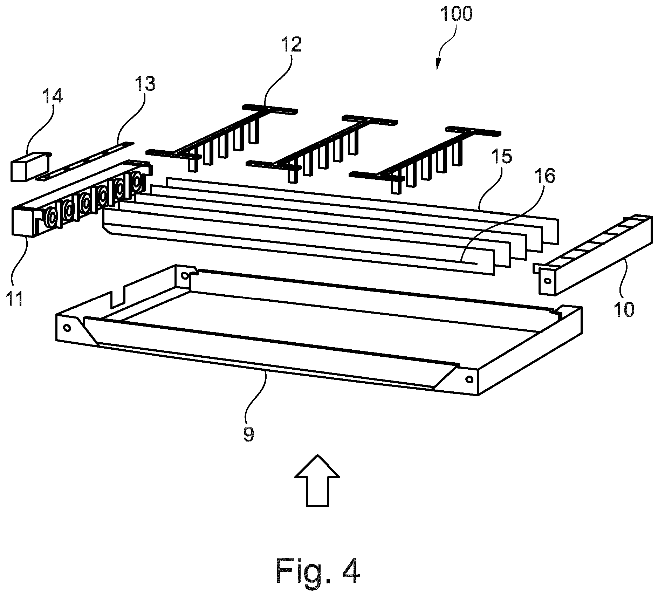

In der Figur 4 ist eine Ausführungsform der Ionisationseinheit 100 der elektrostatischen Filtereinheit 2 in Explosionsansicht gezeigt. In der gezeigten Ausführungsform umfasst die Ionisationseinheit 100 aus einem Innenrahmen 9, zwei Isolationsgehäuseteilen 10, 11, Gegenelektroden 15 und Ionisationselemente 16, die auch als Sprühelektroden bezeichnet werden können. Zudem sind in der gezeigten Ausführungsform Abstandshalter 12 vorgesehen, die die Gegenelektroden 15 in einem Abstand zueinander halten. Weiterhin umfasst die Ionisationseinheit 100 in der gezeigten Ausführungsform einen Hochspannungsübertrager 14 und eine Hochspannungssammelschiene 13.

In den Innenrahmen 9 werden die Isolationsgehäuseteile 10 und 11 eingefügt. Die Isolationsgehäuseteile 10, 11 weisen jeweils eine rechteckige Stabform auf und werden an gegenüberliegenden Enden, insbesondere Längsenden des Innenrahmens 9 in diesen eingelegt. Die Isolationsgehäuseteile 10, 11 weisen jeweils an einer Seite Kontaktöffnungen auf, in die die Ionisationselemente 16 geführt werden. Die Isolationsgehäuseteile 10 und 11 werden so in den Innenrahmen 9 eingebracht, dass die Kontaktöffnungen einander zugewandt sind. Somit können die Ionisationselemente 16, die Drähte darstellen können zwischen den Isolationsgehäuseteilen 10, 11 gespannt werden. Die Gegenelektroden 15, die in der gezeigten Ausführungsform durch Platten gebildet sind, werden in den Innenrahmen 9 und vorzugsweise auch in die Isolationsgehäuseteile 10, 11 eingesteckt. Zudem werden die Abstandshalter 12 aufgebracht, durch die die Gegenelektroden 15 über deren gesamten Länge auf Abstand zueinander gehalten werden. Die Isolationsgehäuseteile, 10, 11 bestehen vorzugsweise aus einem elektrisch isolierenden Werkstoff und weisen vorzugsweise einen Oberflächenwiderstand R >=10¹¹ Ohm auf. Die Isolationsgehäuseteile 10, 11 bestehen vorzugsweise aus Keramik oder Kunststoff.

In einem der Isolationsgehäuseteile 10, 11, in der gezeigten Ausführungsform in dem Isolationsgehäuseteil 11 ist zusätzlich ein Einbauraum vorgesehen. In dem Einbauraum sind der Hochspannungsübertrager 14, die Hochspannungssammelschiene 13 und diverse Verkabelungselemente und Kontakte (nicht gezeigt) vorgesehen, die für die elektrische Kontaktierung der relevanten Einzelteile notwendig sind. Die Ionisationselemente 16 werden anschließend zwischen den Isolationsgehäuseteilen 10, 11 und insbesondere zwichen der Hochspannungssammelschiene 13 des Isolationsgehäuseteiles 11 und dem Isolationsgehäuseteil 10 befestigt.

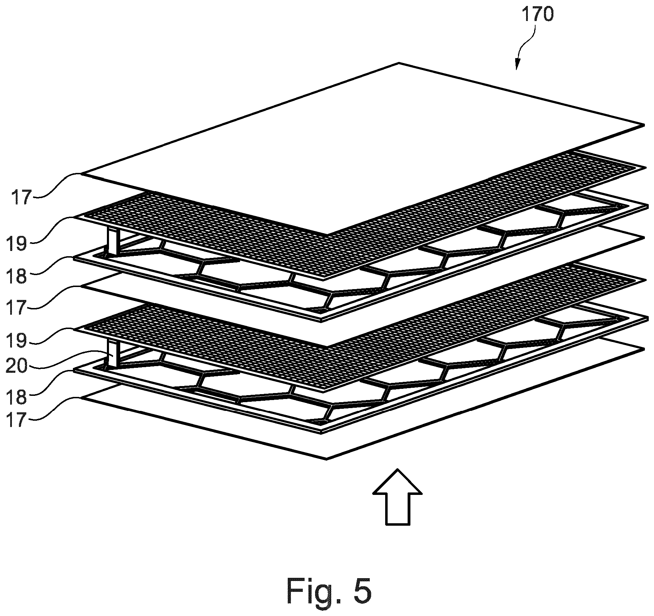

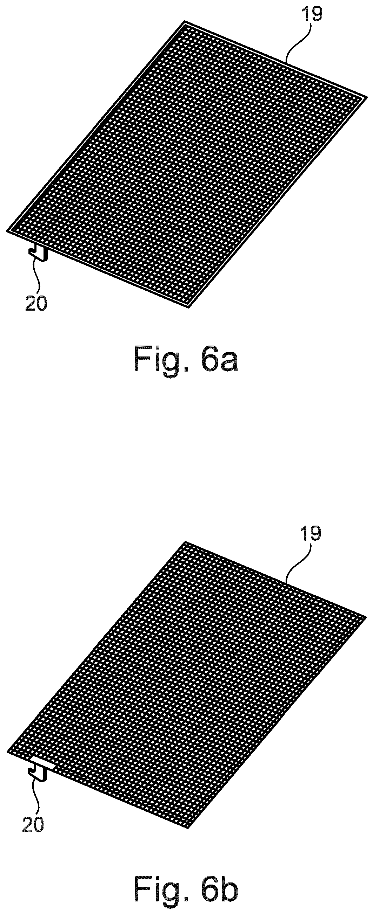

In Figur 5 ist eine Ausführungsform der Abscheideeinheit 170 der elektrostatischen Filtereinheit 2 gezeigt. Die Abscheideeinheit 170 ist aus negativen Niederschlagselektroden 17, Distanzelementen Abscheideeinheit 18 und positiven Niederschlagselektroden 19 aufgebaut. Die Distanzelemente 18 halten die positiven Niederschlagselektroden 19 und negativen Niederschlagselektroden 17 auf definiertem Abstand zueinander. Die positiven Niederschlagselektroden 19 liegen vorzugsweise an Hochspannung U > 1 kV DC (Direct Current) an. Vorzugsweise sind die positiven Niederschlagselektroden 19 sind bis auf die Kontaktstellen 20 mit einem elektrisch isolierenden Überzug vollständig elektrisch isoliert, um elektrische Überschläge und Kurzschlüsse zu unterbinden. Für die Isolierung der positiven Niederschlagselektroden 19 können diese beschichtet werden. Als Beschichtungsverfahren kommen funktionelle Pulverschichtungen, Wirbelsintern, Sol-Gel, Tauchbeschichtung, Emallieren oder Lackieren in Betracht. Als Grundmaterial beziehungsweise Substrat für die positiven Niederschlagselektroden 19 wird vorzugsweise ein elektrisch leitender Werkstoff, insbesondere Metall, verwendet. Besonders bevorzugt können als Substrat für die positiven Niederschlagselektroden 19 die in den Figuren 6a und 6b gezeigten Materialen verwendet werden. In der Ausführungsform nach Figur 6a stellt das Substrat ein Streckmetall und nach Figur 6b ein Schweißgitter dar.

Als Alternative zu Metall für das Substrat der positiven Niederschlagselektroden 19 kommen auch elektrisch leitende oder antistatische Kunststoffe mit einem Oberflächenwiderstand R <= 10¹¹ Ohm in Betracht.

Bei den negativen Niederschlagselektroden 17 handelt es sich ebenfalls um ein luftdurchlässiges Medium. Auch die negativen Niederschlagselektroden 17 bestehen aus einem elektrisch leitenden Material, beispielsweise Metall, oder aus einem antistatischen Werkstoff, beispielsweise Kunststoff mit einem Oberflächenwiderstand R <= 10¹¹ Ohm.

Für die negativen Niederschlagselektroden 17 können beispielsweise Streckmetalle, Drahtgewebe, gewebte Drahtgitter und Kunststoff-Mesh verwendet werden.

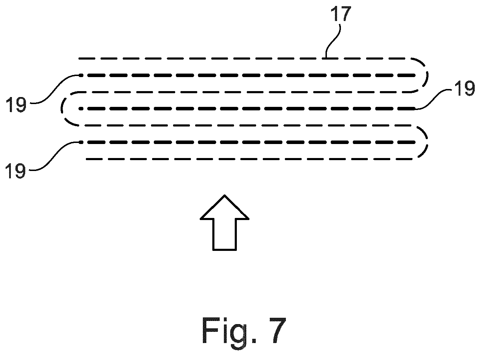

In Figur 7 ist eine alternative Ausführungsform der Niederschlagselektroden 17, 19 der Abscheideeinheit 170 gezeigt. Bei der in Figur 7 gezeigten Ausführungsform besteht die negative Niederschlagselektrode 17 aus einem Einzelteil, die schlangenförmig oder mäanderförmig zwischen den positiven Niederschlagselektroden 19 angeordnet wird. Diese Ausführungsform hat den Vorteil, dass die einzelnen negativen Niederschlagselektroden 17 nicht separat mit einander elektrisch kontaktiert werden müssen über Kontakte oder sonstiges.

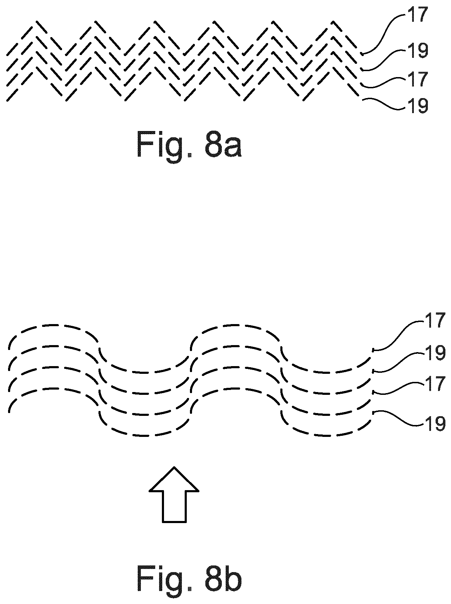

In Figuren 8a und 8b sind weitere Ausführungsformen der Niederschlagselektroden der Abscheideeinheit 170 gezeigt. In dieser Ausführungsform weisen die Niederschlagselektroden 17, 19 jeweils eine plissierte oder gewellte Struktur auf. Diese Maßnahme dient einerseits der Druckverlustreduzierung Delta p [Pa] beim Durchströmen des Filtermediums (der Abscheideeinheit 170) und andererseits einer Steigerung der Filtereffizienz bei sonst gleichbleibenden Randbedingungen. Insbesondere kann die Fläche der Niederschlagselektroden 17, 19 bei gleicher Breite und Länge des Filterrahmens 40 vergrößert werden.

In jeder der Ausführungsformen der Niederschlagselektroden 17, 19 kann diese jeweils aus einer einzigen Lage bestehen. Alternativ kann aber die positive Niederschlagselektrode 19 aus mehreren Lagen n>=1 aufgebaut sein. Dasselbe gibt auch für die negative Niederschlagselektrode 17.

Die Anzahl an positiven Niederschlagselektroden 19 je Filtermodul entspricht >= 1. Die Anzahl an negativen Niederschlagselektroden 17 je Filtermodul entspricht ebenfalls >= 1. Zur Steigerung der Filtereffizienz können Distanzelemente 18 verwendet werden. Alternativ können diese auch ausgelassen werden und die positiven und negativen Niederschlagselektroden 17, 19 direkt aufeinandergestapelt werden. Jedoch bewirken die Distanzelemente 18 eine Performance-Steigerung der Filtereffizienz.

Vorzugsweise ist die Reihenfolge der positiven und negativen Niederschlagselektroden 17, 19, wie beispielsweise in Figur 5 gezeigt, alternierend. Bei der in Strömungsrichtung ersten und letzten Niederschlagselektrode kann es sich sowohl jeweils entweder um eine positive als auch eine negative Niederschlagselektrode handeln. Dies bedeutet, dass die erste Lage beziehungsweise Elektrode positiv oder negativ sein kann und die letzte Lage beziehungsweise Elektrode ebenfalls positiv oder negativ sein kann.

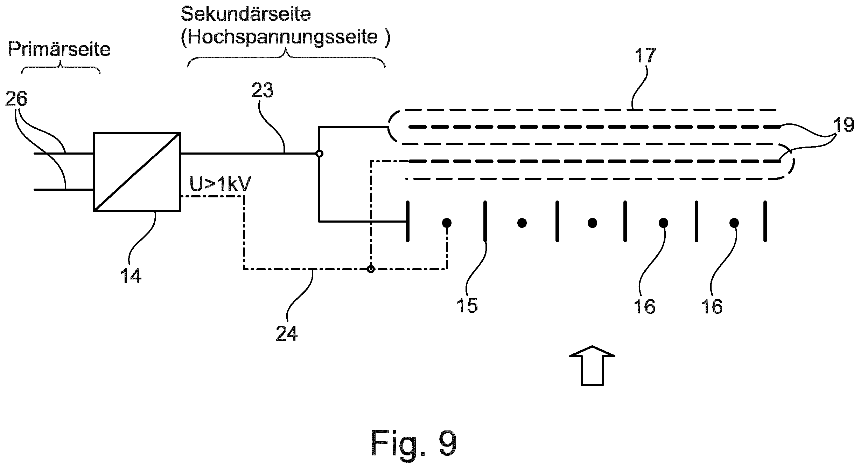

Die elektrische Verkabelung der elektrostatische Filtereinheit 2 ist in Figur 9 schematisch dargestellt. Über Kontaktstellen beziehungsweise Anschlüsse der geringen Spannung 26 auf der Primärseite des Hochspannungsübertragers 14 erfolgt die elektrische Leistungsversorgung mit Kleinspannung (<= 50V AC; <= 120 V DC) oder Niederspannung (<= 1000V AC; <= 1500 V DC).

Die Leistungsversorgung erfolgt über Kontaktelemente (nicht gezeigt) zwischen jeder einzelnen elektrostatische Filtereinheit 2 und dem Dunstabzug beziehungsweise Luftreinigungsvorrichtung 1. Die elektrische Kontaktierung erfolgt idealerweise zwischen der Auflagefläche des Filterrahmens Rückseite 5 und der Luftreinigungsvorrichtung 1 im Bereich der Ansaugöffnung. Die Auflagefläche des Filterrahmens Rückseite 5 ist die in Figur 2 nach oben gerichtete Fläche des Filterrahmens Rückseite 5. Alternativ kann die elektrische Kontaktschnittstelle an jeder beliebigen Stelle der Außenfläche der Filterrahmenelemente 4 und 5 erfolgen.

Auf der Sekundärseite ist die Hochspannungsleitung 24 mit U > 1kV DC über die Hochspannungssammelschiene 13 (s. Figur 4) mit den Ionisationselementen 16 verbunden und weiterhin mit allen positiven Niederschlagselektroden 19. Die Rückleitung 23 der Sekundärseite ist verbunden mit allen Gegenelektroden 15 und mit der / den negativen Niederschlagselektroden 17. Der Filterrahmen Vorderseite 4, der Filterrahmen Rückseite 5 und Filterlage der Vorfilterung 8 sind ebenfalls mit der Rückleitung 23 verbunden und befinden sich auf demselben elektrischen Potential.

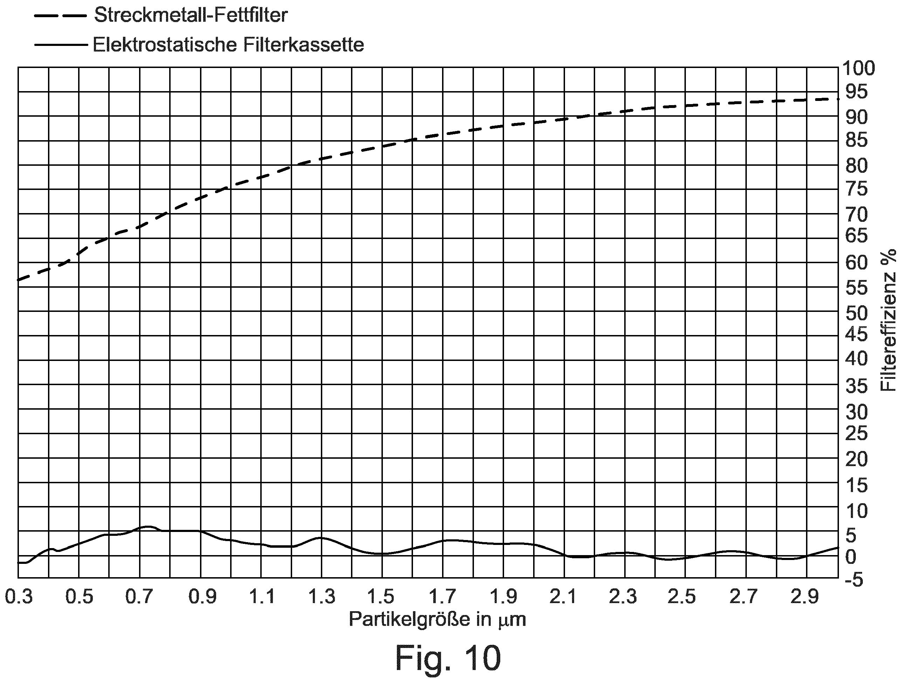

In Figur 10 ist für den Partikelgrößenbereich 0,3 <= d_{hyd} <= 3µm die Filtereffizienz eines Streckmetallfilters und einer Ausführungsform der erfindungsgemäßen elektrostatischen Filtereinheit 2 für eine mittlere Strömungsgeschwindigkeit c < 1m/s dargestellt. Wie man aus dem Diagramm ablesen kann, weist die erfindungsgemäße elektrostatische Filtereinheit 2 eine signifikant bessere Filtereffizienz auf als der klassische Streckmetallfilter eines Dunstabzugs.

Die vorliegende Erfindung weist eine Reihe von Vorteilen auf.

Die vollständig umschlossene, elektrisch leitende oder antistatische Hülle mit vorzugsweise einem Oberflächenwiderstand R <= 10¹¹ Ohm, die der elektrischen Abschirmung dient, schützt den Benutzer/Konsumenten und seine Umwelt vor elektrischer Gefährdung. Alle unter Hochspannung liegenden Einzelteile sind durch diesen konstruktiven Aufbau vollständig durch leitende oder antistatische Elemente gekapselt. Eine elektrostatische Aufladung der äußersten für den Konsumenten unmittelbar berührbaren Einzelteile, insbesondere Filterrahmen und Eingreifschutz, wird unterbunden.

Erfindungsgemäß weist die Filtereinheit mindestens ein Schutzelement für zumindest eines der Elemente der Ionisationseinheit auf, wobei zumindest ein Schutzelement eine Eingreifschutzvorrichtung darstellt. Dadurch können elektrostatische Filtermechanismen verwendet werden, ohne, dass es zu einer Gefahr für den Benutzer kommt. Hierdurch kann eine signifikant bessere Filtereffizienz für flüssige und feste Partikel mit einem hydraulischen Durchmesser d_{hyd} < 100µm gegenüber den klassischen Dunstabzugshauben-Fettfiltern, speziell für niedrige Strömungsgeschwindigkeiten c < 2 m/s erzielt werden. Die verbesserte Filtereffizienz ist insbesondere bei der Ausführungsform der elektrostatische Filtereinheit gegeben, bei der poröse Niederschlagselektroden verwendet werden und dadurch die weiterhin wirkenden mechanischen Abscheidemechanismen mittels Diffusions-, Sperr- und Massenträgheitseffekt der elektrostatischen Filtermechanismen überlagert wird. Aufgrund der signifikant besseren Filtereffizienz, speziell bei niedrigen Strömungsgeschwindigkeiten für c < 2m/s, bleiben die dahinterliegenden Elemente des Dunstabzuges (Lüfter, Gehäuse, Geruchsfilter) frei von Öl- und Schmutzansammlungen. Speziell für umluftbetriebene Dunstabzüge, welche in Strömungsrichtung einen hinter dem Fettfilter nachgeschalteten Geruchsfilter (Aktivkohle oder Zeolithfilter) vorweisen, bleibt der Lebensdauer (Standzeit) für diesen Umluftfilter länger erhalten beziehungsweise wird dadurch verlängert, da dieser in diesem Fall nicht mit feinsten Partikeln kontaminiert wird und sich dadurch zusetzt. Eine solche elektrostatische Filterkassette weist im Gegensatz zum klassischen Fettfilter eines Dunstabzugs bei gleicher Filtereffizienz einen deutlich geringeren Druckverlust Delta p auf.

Indem erfindungsgemäß ein Schutzelement vorgesehen ist, das beispielsweise ein Isolierungsgehäuseteil sein kann, kann der Hochspannungsträger in die elektrostatische Filtereinheit implementiert werden, ohne, dass dieser eine Gefahr für den Benutzer darstellt. Da zudem die elektrostatische Filtereinheit über Kontakte, mit Kleinspannung (<= 50V AC; <= 120 V DC) oder Niederspannung (<= 1000 V AC; <=1500 V DC) mit elektrischer Energie von der Luftreinigungsvorrichtung versorgt werden kann wird weiterhin die elektrische Gefährdung für den Benutzer/Konsumenten gesenkt.

Schließlich senkt die bevorzugte Verwendung von luftdurchlässigen "porösen" Niederschlagselektroden anstelle von Plattenpaketen (Plattenpaket entspricht dem Aufbau klassischer Elektrofilter für die Abscheidung) die Komplexität und den Kostenpunkt des Gesamtsystems, weil weniger Einzelteile eingesetzt werden. Durch die Verwendung einer luftdurchlässigen (porösen), isolierenden Niederschlagselektrode entsteht ein feinmaschiges elektrostatisches Feld mit hoher elektrischer Feldstärke. Es ermöglicht eine hohe Abscheideleistung bei sehr geringem Bauraum im Vergleich zum klassischen Plattenelektrofilter nach dem Penney-Prinzip. Durch die vorliegende Erfindung kann diese hohe elektrische Feldstärke genutzt werden, ohne, dass der Benutzer gefährdet wird.

Zudem wird durch die Verwendung luftdurchlässiger Niederschlagselektroden im Gegensatz zu klassischen Elektrofilter mit Plattenabscheidung, die aufgrund ihrer Bauart nur über geringes Speichervermögen verfügen, ein größeres Speichervermögen bereitgestellt. Gefilterte/abgeschiedene Partikel werden bei klassischen Elektrofiltern mit Plattenabscheidung an der Oberfläche der luftundurchlässigen Abscheideplatte gesammelt. Bei größeren Ansammlungen von Partikeln ist die Haltefähigkeit der Oberfläche erschöpft. Es kommt zu Ablösung der gefilterten/abgeschiedenen Partikel. Diese werden dann beispielsweise durch den Luftstrom wieder mitgerissen. Durch die Verwendung von luftdurchlässigen/porösen Niederschlagselektroden entstehen Einlagerungs- beziehungsweise Speicherstrukturen. Ein Ablösen von bereits abgeschiedenen Partikeln wird stark reduziert.

Schließlich kann aufgrund der vorzugsweise vorgesehenen elektrisch isolierenden Beschichtung der positiven Niederschlagselektroden ein direktes Aufeinanderliegen der Elektroden ermöglicht werden, ohne, dass das elektrische Feld aufgrund von elektrischen Kriechströmen und Funkenüberschlägen zwischen positiven und negativen Niederschlagselektroden zusammenbricht. Die elektrostatische Filtereinheit weist dadurch eine bessere Kriechstromfestigkeit auf.

### Bezugszeichenliste

- 1: Luftreinigungsvorrichtung
- 2: elektrostatische Filtereinheit
- 3: Luftauslassstutzen von Luftreinigungsvorrichtung
- 40: Filterrahmen
- 4: Filterrahmen Vorderseite
- 5: Filterrahmen Rückseite
- 60: Schutzelement
- 6: Schutzlage
- 7: Distanzelement Eingreifschutz
- 8: Filterlage der Vorfilterung
- 9: Innenrahmen
- 100: Ionisationseinheit
- 10: Isolationsgehäuseteil
- 11: Isolationsgehäuseteil incl. Einbauraum
- 12: Abstandshalter
- 13: Hochspannungssammelschiene
- 14: Hochspannungsübertrager
- 15: Gegenelektrode
- 16: lonisationselement/Sprühelektrode
- 170: Abscheideeinheit
- 17: negative Niederschlagselektroden
- 18: Distanzelement Abscheideeinheit
- 19: positive Niederschlagselektrode
- 20: Kontakt der positiven Niederschlagselektroden
- 23: Rückleitung der Sekundärseite/Anschluss der negativen Niederschlagselektrode
- 24: Hochspannungsanschluss/Hochspannungsleitung der Sekundärseite
- 26: Anschluss der geringen Spannung

- Dhyd =: hydraulischer Durchmesser

## Patentansprüche

1. Dunstabzugshaube, die mindestens eine elektrostatische Filtereinheit aufweist, wobei die Filtereinheit (2) eine Ionisationseinheit (100) und eine Abscheideeinheit (170) mit mindestens zwei Niederschlagselektroden (17, 19) umfasst, wobei die mindestens zwei Niederschlagselektroden (17, 19) luftdurchlässig sind und die Filtereinheit (2) mindestens ein Schutzelement für zumindest eines der Elemente der Ionisationseinheit (100) aufweist, zumindest ein Schutzelement (60) eine Eingreifschutzvorrichtung darstellt, die mindestens eine Lage aufweist und der Ionisationseinheit (100) in Strömungsrichtung vorgeschaltet ist und mindestens eine Lage einen Vorfilter (8) darstellt, **dadurch gekennzeichnet, dass** die mindestens zwei Niederschlagselektroden (17,19) aus einem luftdurchlässigen Material bestehen und senkrecht zu der Strömungsrichtung der Luft durch die Ionisationseinheit (100) liegen.

2. Dunstabzugshaube nach Anspruch 1, wobei das Schutzelement (60) ein Abschirmelement und / oder ein Schutzelement gegen Krafteinwirkung darstellt.

3. Dunstabzugshaube nach einem der Ansprüche 1 oder 2, wobei die Eingreifschutzvorrichtung mindestens eine Schutzlage (6) und ein Distanzelement (7) aufweist.

4. Dunstabzugshaube nach einem der Ansprüche 1 bis 3, **dadurch gekennzeichnet, dass** das Schutzelement (60) zumindest teilweise aus einem elektrisch leitenden oder antistatisches Element mit einen Oberflächenwiderstand R <= 10¹¹ Ohm besteht.

5. Dunstabzugshaube nach einem der Ansprüche 1 bis 4, wobei mindestens ein Schutzelement (60) zumindest einen Teil (10,11) eines Gehäuses für die Ionisationseinheit (100) darstellt.

6. Dunstabzugshaube nach Anspruch 5, wobei die Filtereinheit (2) einen Hochspannungsübertrager (14) aufweist und der Hochspannungsübertrager (14) in einem der Gehäuseteile (10, 11) integriert ist.

7. Dunstabzugshaube nach Anspruch 6, **dadurch gekennzeichnet, dass** der Vorfilter (8) mit der Rückführung der Sekundärseite des Hochspannungsübertragers (14) verbunden ist.

8. Dunstabzugshaube nach Anspruch 7, **dadurch gekennzeichnet, dass** die elektrostatische Filtereinheit (2) einen Filterrahmen (40) aufweist und der Filterrahmen (40) mit der Rückführung der Sekundärseite des Hochspannungsübertragers (14) verbunden ist.

9. Dunstabzugshaube nach einem der Ansprüche 1 bis 8, wobei mindestens eine der Niederschlagselektroden (17, 19), insbesondere die positiven Niederschlagselektroden (19), eine Isolierungsbeschichtung aufweist.

10. Dunstabzugshaube nach einem der Ansprüche 1 bis 9, wobei die Dunstabzugshaube mindestens eine Kontaktstelle für den Kontakt mit einem Hochspannungsübertrager (14) der Filtereinheit (2) aufweist.

## Claims

1. Extractor hood, which has at least one electrostatic filter unit, wherein the filter unit (2) comprises an ionisation unit (100) and a separation unit (170) with at least two collecting electrodes (17, 19), wherein the at least two collecting electrodes (17, 19) are air-permeable and the filter unit (2) has at least one protection element for at least one of the elements of the ionisation unit (100), at least one protection element (60) takes the form of an intervention protection device which has at least one layer and is connected upstream of the ionisation unit (100) in the direction of flow and at least one layer takes the form of a prefilter (8), **characterised in that** the at least two collecting electrodes (17, 19) consist of an air-permeable material and lie perpendicular to the direction of flow of the air through the ionisation unit (100).

2. Extractor hood according to claim 1, wherein the protection element (60) takes the form of a screening element and / or a protection element against the effect of force.

3. Extractor hood according to one of claims 1 or 2, wherein the intervention protection device has at least one protection layer (6) and a spacer element (7).

4. Extractor hood according to one of claims 1 to 3, **characterised in that** the protection element (60) consists at least partially of an electrically conductive or antistatic element having a surface resistance R <= 10¹¹ Ohms.

5. Extractor hood according to one of claims 1 to 4, wherein at least one protection element (60) takes the form of at least part (10, 11) of a housing for the ionisation unit (100).

6. Extractor hood according to claim 5, wherein the filter unit (2) has a high-voltage transformer (14) and the high-voltage transformer (14) is integrated in one of the housing parts (10, 11).

7. Extractor hood according to claim 6, **characterised in that** the prefilter (8) is connected to the return of the secondary side of the high-voltage transformer (14).

8. Extractor hood according to claim 7, **characterised in that** the electrostatic filter unit (2) has a filter frame (40) and the filter frame (40) is connected to the return of the secondary side of the high-voltage transformer (14).

9. Extractor hood according to one of claims 1 to 8, wherein at least one of the collecting electrodes (17, 19), in particular the positive collecting electrodes (19), have an insulation coating.

10. Extractor hood according to one of claims 1 to 9, wherein the extractor hood has at least one contact point for the contact to a high-voltage transformer (14) of the filter unit (2).

## Revendications

1. Hotte aspirante qui présente au moins une unité filtrante électrostatique, dans laquelle l'unité filtrante (2) comprend une unité d'ionisation (100) et une unité de séparation (170) avec au moins deux électrodes de précipitation (17, 19), dans laquelle les au moins deux électrodes de précipitation (17, 19) sont perméables à l'air et l'unité filtrante (2) présente au moins un élément de protection pour au moins un des éléments de l'unité d'ionisation (100), au moins un élément de protection (60) constitue un dispositif de protection contre les interventions, qui présente au moins une couche et est disposé en amont de l'unité d'ionisation (100) dans le sens d'écoulement, et au moins une couche constitue un préfiltre (8), **caractérisée en ce que** les au moins deux électrodes de précipitation (17, 19) sont composées d'un matériau perméable à l'air et sont disposées perpendiculairement au sens d'écoulement de l'air à travers l'unité d'ionisation (100).

2. Hotte aspirante selon la revendication 1, dans laquelle l'élément de protection (60) constitue un élément de blindage et/ou un élément de protection contre l'action d'une force.

3. Hotte aspirante selon l'une des revendications 1 ou 2, dans laquelle le dispositif de protection contre les interventions présente au moins une couche de protection (6) et un élément d'espacement (7).

4. Hotte aspirante selon l'une des revendications 1 à 3, **caractérisée en ce que** l'élément de protection (60) est au moins en partie composé d'un élément électriquement conducteur ou antistatique ayant une résistance superficielle de R <= 10¹¹ ohms.

5. Hotte aspirante selon l'une des revendications 1 à 4, dans laquelle au moins un élément de protection (60) constitue au moins une partie (10, 11) d'un boîtier pour l'unité d'ionisation (100).

6. Hotte aspirante selon la revendication 5, dans laquelle l'unité filtrante (2) présente un transformateur haute tension (14) et le transformateur haute tension (14) est intégré à l'une des parties de boîtier (10, 11).

7. Hotte aspirante selon la revendication 6, **caractérisée en ce que** le préfiltre (8) est relié au retour du côté secondaire du transformateur haute tension (14).

8. Hotte aspirante selon la revendication 7, **caractérisée en ce que** l'unité filtrante électrostatique (2) présente un cadre de filtre (40) et le cadre de filtre (40) est relié au retour du côté secondaire du transformateur haute tension (14).

9. Hotte aspirante selon l'une des revendications 1 à 8, dans laquelle au moins une des électrodes de précipitation (17, 19), en particulier les électrodes de précipitation positives (19), comporte un revêtement isolant.

10. Hotte aspirante selon l'une des revendications 1 à 9, dans laquelle la hotte aspirante présente au moins un point de contact pour le contact avec un transformateur haute tension (14) de l'unité filtrante (2).
